# EUROPEAN PATENT APPLICATION

(11) **EP 3 847 958 A1**
(43) Date of publication of application: **14.07.2021**
(21) Application number: 18932602.8
(22) Date of filing: 04.09.2018
(51) Int. Cl.: A61B 5/0402

(54) **ARRHYTHMIA DETECTION METHOD AND APPARATUS, ELECTRONIC DEVICE, AND COMPUTER STORAGE MEDIUM**

(71) Applicant: Shenzhen Institutes of Advanced Technology, Shenzhen, Guangdong 518055 (CN)
(72) Inventor: YAO, Qihang, Shenzhen, Guangdong 518055 (CN); LI, Ye, Shenzhen, Guangdong 518055 (CN); FAN, Xiaomao, Shenzhen, Guangdong 518055 (CN); CAI, Yunpeng, Shenzhen, Guangdong 518055 (CN)
(74) Representative: de Arpe Fernandez, Manuel
(86) International application number: PCT/CN2018/104002
(87) International publication number: WO 2020/047750

(57) **Abstract**

This application provides an arrhythmia detection method and apparatus, an electronic device, and a computer storage medium, which relate to the technical field of electrocardiogram (ECG) detection. The method includes: obtaining an ECG signal to be detected; inputting the ECG signal to a pre-established arrhythmia detection model, the arrhythmia detection model including a convolutional neural network and a recurrent neural network that are connected in sequence; and detecting the ECG signal by the arrhythmia detection model to obtain a detection result corresponding to the ECG signal, the detection result including an arrhythmia type. This application can perform arrhythmia detection on ECG signals of different lengths, thus effectively improving the versatility of arrhythmia detection.

## Description

### TECHNICAL FIELD

This application relates to the technical field of electrocardiogram (ECG) detection, and more particularly relates to an arrhythmia detection method and apparatus, an electronic device, and a computer storage medium.

### BACKGROUND

Arrhythmia refers to abnormal heart rhythm, heart rate, or activation sequence caused by heartbeat and conduction disorders, and has various types such as tachycardia, bradycardia, irregular heartbeat, cardiac arrest, fibrillation, and the like. Detection of arrhythmia is of great significance for the prevention of cardiovascular diseases.

The traditional method for arrhythmia detection is manual detection, that is, a doctor with professional knowledge analyzes the electrocardiogram to determine the type of arrhythmia. With the development of artificial intelligence (AI), the applications of convolutional neural network (CNN) models to automatically detect arrhythmia types are emerging. However, the existing convolutional neural network models can only perform arrhythmia detection on ECG signals with a fixed length, and are difficult to apply to ECG signals of different lengths. That is, they are restricted by the different signal lengths commonly present in ECG data, resulting in poor versatility.

### SUMMARY

In view of the above, it is an object of this application to provide an arrhythmia detection method and apparatus, an electronic device, and a computer storage medium, which can perform arrhythmia detection on ECG signals of different lengths, thus effectively improving the versatility of arrhythmia detection.

In order to achieve at least one of the above objectives, this application adopts the following technical solutions.

According to a first aspect, there is provided an arrhythmia detection method, which is executed by a processor of an electronic device, the method including: obtaining an electrocardiogram (ECG) signal to be detected; inputting the ECG signal into a pre-established arrhythmia detection model, where the arrhythmia detection model includes a convolutional neural network (CNN) and a recurrent neural network (RNN) that are sequentially connected; detecting the ECG signal by the arrhythmia detection model to obtain a detection result corresponding to the ECG signal, the detection result including an arrhythmia type.

In an optional embodiment of the present application, the operation of detecting the ECG signal by the arrhythmia detection model to obtain the detection result corresponding to the ECG signal includes: performing feature extraction on the input ECG signal by the convolutional neural network to obtain feature information of the electrocardiogram signal, and inputting the feature information into the recurrent neural network; and classifying the input feature information by the recurrent neural network to determine the arrhythmia type of the ECG signal.

In an optional embodiment of the present application, the operation of performing feature extraction on the input ECG signal by the convolutional neural network to obtain feature information of the ECG signal includes: performing iterated convolution treatment and nonlinear transformation treatment on the ECG signal by the convolutional neural network to obtain a characteristic temporal sequence, the characteristic temporal sequence including characteristic segments that are respectively extracted from multiple segments of the ECG signal and that are arranged in chronological order.

In an optional embodiment of the present application, the operation of classifying the input feature information by the recurrent neural network to determine the arrhythmia type of the ECG signal includes: performing iterative processing on each of the characteristic segments in the characteristic temporal sequence using the recurrent neural network to determine the final state of the characteristic temporal sequence, and classifying the final state of the characteristic temporal sequence and determining the arrhythmia type of the ECG signal.

In an optional embodiment of the present application, the training process of the arrhythmia detection model includes: obtaining a training signal; inputting the training signal into the arrhythmia detection model to be trained, and calculating the loss function value by a loss function of the arrhythmia detection model; training the parameters of the arrhythmia detection model using a backpropagation algorithm based on the loss function value, until the loss function value converges to a preset value.

In an optional embodiment of the present application, the operation of obtaining the training signal includes: obtaining an original ECG signal; preprocessing the original ECG signal to generate a training signal, the preprocessing includes one or more selected from the group consisting of noise treatment, transverse stretching treatment, transverse compression treatment, and partial masking treatment.

In an optional embodiment of the present application, the preprocessing includes noise treatment, and the operation of preprocessing the original ECG signal to generate the training signal includes: adding a random noise corresponding to the amplitude of a preset signal-to-noise ratio to the original ECG signal to obtain the training signal.

In an optional embodiment of the present application, the preprocessing includes transverse stretching treatment and/or transverse compression treatment, and the operation of preprocessing the original ECG signal to generate the training signal includes: performing transverse stretching treatment and/or transverse compression treatment on the original ECG signal according to a preset ratio to obtain the training signal.

In an optional embodiment of the present application, the preprocessing includes partial masking treatment, and the operation of preprocessing the original ECG signal to generate the training signal includes: randomly selecting a signal segment having a duration less than a preset duration from the original ECG signal; and setting the selected signal segment in the original ECG signal to zero to obtain the training signal.

In an optional embodiment of the present application, the training process of the arrhythmia detection model adopts one or more stable training methods selected from the group consisting of a regularization method, a network connection structure random removal method, and a label interference method.

In an optional embodiment of the present application, the convolutional neural network includes a plurality of convolutional layers and a plurality of pooling layers. Each of the convolutional layers includes a convolution unit, a nonlinear transformation unit, and a batch normalization unit that are connected in sequence. The recurrent neural network includes a plurality of long- and short-term memory units, where each of the long- and short-term memory units includes a forget gate structure, an update gate structure, and an output gate structure.

According to a second aspect, the present application further provides an arrhythmia detection apparatus, which is arranged on the processor side of an electronic device, the arrhythmia detection apparatus including: a signal acquisition module configured to obtain an electrocardiogram (ECG) signal to be detected; a model input module configured to input the ECG signal into a pre-established arrhythmia detection model, where the arrhythmia detection model includes a convolutional neural network (CNN) and a recurrent neural network (RNN) that are sequentially connected; and a model detection module configured to detect the ECG signal by the arrhythmia detection model to obtain a detection result corresponding to the ECG signal, the detection result including the arrhythmia type.

In an optional embodiment of the present application, the model detection module is implemented as: a feature extraction unit configured for performing feature extraction on the input ECG signal by the convolutional neural network to obtain feature information of the electrocardiogram signal, and inputting the feature information into the recurrent neural network; and a type determination unit configured for classifying the input feature information by the recurrent neural network to determine the arrhythmia type of the ECG signal.

In an optional embodiment of the present application, the feature extraction unit is configured for performing iterated convolution treatment and nonlinear transformation treatment on the ECG signal by the convolutional neural network to obtain a characteristic temporal sequence, the characteristic temporal sequence including multiple characteristic segments that are respectively extracted from multiple segments of the ECG signal and that are arranged in chronological order.

In an optional embodiment of the present application, the type determination unit is configured for performing iterative processing on each of the characteristic segments in the characteristic temporal sequence using the recurrent neural network to determine the state of the characteristic temporal sequence, and classifying the final state of the characteristic temporal sequence and determining the arrhythmia type of the ECG signal.

In an optional embodiment of the present application, the arrhythmia detection apparatus further includes: a training signal acquisition module configured for obtaining a training signal; a loss value calculation module configured for inputting the training signal into the arrhythmia detection model to be trained, and calculating the loss function value by a preset loss function of the arrhythmia detection model; and a training module configured for training the parameters of the arrhythmia detection model using a backpropagation algorithm based on the loss function value, until the loss function value converges to a preset value.

In an optional embodiment of the present application, the training signal acquisition module is configured for obtaining an original ECG signal, and preprocessing the original ECG signal to generate a training signal, the preprocessing includes one or more selected from the group consisting of noise treatment, transverse stretching treatment, transverse compression treatment, and partial masking treatment.

In an optional embodiment of the present application, the convolutional neural network includes a plurality of convolutional layers and a plurality of multiple pooling layers. Each of the convolutional layers includes a convolution unit, a nonlinear transformation unit, and a batch normalization unit that are connected in sequence. The recurrent neural network includes a plurality of long- and short-term memory units, where each of the long- and short-term memory units includes a forget gate structure, an update gate structure, and an output gate structure.

According to a third aspect, there is provided an electronic device that includes a memory and a processor, where the memory is configured to store a program that supports the processor to execute any one of the methods according to the first aspect, and the processor is configured to execute the program stored in the memory.

According to a fourth aspect, there is provided a computer storage medium configured for storing computer software instructions used by any arrhythmia detection apparatus described in the second aspect.

According to the above-described arrhythmia detection method and apparatus, electronic device, and computer storage medium, the ECG signal to be detected is input into the pre-established arrhythmia detection model, and the detection result including the arrhythmia type can be obtained through the arrhythmia detection model. In comparison with the fact that the traditional convolutional neural network models in the related art can only perform arrhythmia detection on ECG signals with a fixed length thus resulting in significant limitations, the arrhythmia detection model proposed in this application combines the convolutional neural network and the recurrent neural network, and so can detect ECG signals of different lengths depending on the characteristics of recurrent neural networks, thus effectively improving the versatility of arrhythmia detection.

Other features and advantages of this application will be described in the following specification, or some of the features and advantages can be unambiguously inferred or determined from the specification, or can be learned by implementing the above-mentioned technology of this application.

In order to render the above-mentioned objectives, features and advantages of the present application more evident and understandable, some illustrative embodiments will be described below in further detail in connection with the accompanying drawings.

### BRIEF DESCRIPTION OF DRAWINGS

To better illustrate the technical solutions in specific embodiments of this application or those in the related art, the drawings that need to be used in the description of the embodiments or the related art will be briefly introduced below. Apparently, the drawings in the following description merely represent some embodiments of this application, and for those having ordinary skill in the art, other drawings may also be obtained based on these drawings without investing creative efforts.
FIG. 1 shows a flowchart illustrating an arrhythmia detection method provided by this application.
FIG. 2 shows a schematic diagram illustrating an arrhythmia detection model provided by this application.
FIG. 3 shows a flowchart illustrating another arrhythmia detection model provided by this application.
FIG. 4 shows a flowchart illustrating another arrhythmia detection method provided by this application.
FIG. 5 shows a flowchart illustrating a training method for an arrhythmia detection model according to this application.
FIG. 6 shows a block diagram illustrating an arrhythmia detection apparatus provided by this application.
FIG. 7 shows a schematic diagram illustrating the configuration of an electronic device provided by this application.

### DETAILED DESCRIPTION

For a better understanding of the objectives, technical solutions, and advantages of the embodiments of this application, hereinafter technical solution in accordance with the present application will be described in a definite and comprehensive manner in connection with the accompanying drawings. It is evident that the embodiments described herein represent merely a part rather than all of the embodiments in accordance with this application. All other embodiments obtained by a person having ordinary skill in the art based on the embodiments disclosed herein without making creative efforts shall all fall in the scope of protection of this application.

The existing automatic arrhythmia detection methods use a convolutional neural network model, but the ECG signals that such convolutional neural network models are able to detect have a fixed length, that is, the length of the ECG signal input into the model needs to be somewhat limited. This is because the traditional convolutional neural network model usually relies on the fully connected layer of the convolutional neural network to output the result, while the number of neurons in the fully connected layer needs to correspond to the input signal, and so the convolutional neural network model has some requirements on the length of the input signal due to the limitation of the fully connected layer. As such, the convolutional neural network model requires a fixed segmentation of the ECG signal to obtain a fixed length model input; this method however seriously affects the accuracy of arrhythmia detection. In addition, when the input signal of the convolutional neural network model is limited to close to a waveform length, although the convolutional neural network model has recognition capability with respect to arrhythmias with obvious abnormal waveforms, it is powerless for arrhythmias that are mainly manifested in rhythm characteristics. Furthermore, this method needs to label single waveforms, so the labeling cost is too high and it does not have good applicability. If the input signal of the convolutional neural network model is expanded to the record length level, it will be limited by the problem of different signal lengths that are commonly present in ECG data sets. The scale of the convolutional neural network model needs to be increased correspondingly with the length of the input signal, which will also cause problems such as high computational overhead and over-fitting.

In view of this, this application provides an arrhythmia detection method and apparatus, an electronic device, and a computer storage medium, which can perform arrhythmia detection on ECG signals of different lengths (i.e., ECG signals of variable lengths), and so can effectively improve at least one of the above-mentioned problems. Hereinafter this application will be described in greater detail.

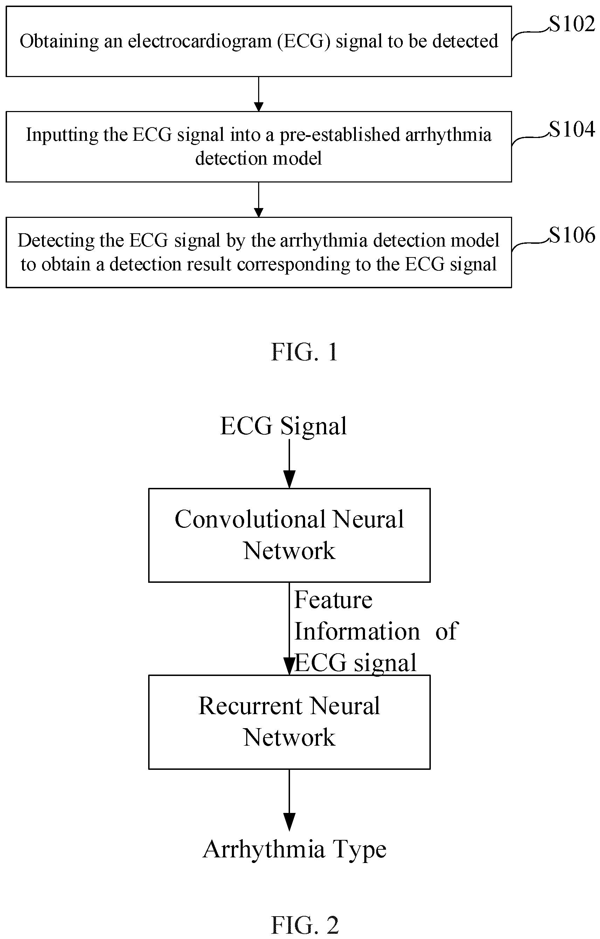

FIG. 1 shows a flowchart illustrating an arrhythmia detection method. Each step of this method is executed by the processor of an electronic device, which may be a computer, a mobile phone, a wearable heart monitoring device, or the like. The method may include the following operations.

In S102, the method includes obtaining an electrocardiogram (ECG) signal to be detected. The ECG signal may be an ECG signal of a user to be detected that is obtained in real time by the processor of the electronic device, or it may be the ECG signal stored in a designated location and called by the processor from the designated location.

In S104, the method includes inputting the ECG signal into a pre-established arrhythmia detection model, where the arrhythmia detection model may include a convolutional neural network and a recurrent neural network that are connected in sequence. Referring to the schematic diagram of an arrhythmia detection model shown in FIG. 2, a simple structure of an arrhythmia detection model is illustrated. In one implementation, the convolutional neural network (CNN) may be used to perform feature extraction on the input ECG signal to obtain the feature information of the ECG signal, and the feature information may in turn be inputted to the Recurrent Neural Network (RNN). Then the recurrent neural network may be used to classify the input feature information to determine the arrhythmia type of the ECG signal.

The above-described arrhythmia detection model proposed in this embodiment integrates CNN and RNN, where CNN is used to enhance original signal features and reduce noise through convolution operations, and RNN is used to introduce a directional loop, which may be mainly used to process sequence data, and can deal with the problem of correlations between inputs, so as to perform better classification.

In S106, the method includes detecting the ECG signal by the arrhythmia detection model to obtain a detection result corresponding to the ECG signal, the detection result including the arrhythmia type. Arrhythmia includes various types, such as sinus tachycardia, sinus bradycardia, premature beats, paroxysmal supraventricular tachycardia, paroxysmal ventricular tachycardia, atrial flutter, atrial fibrillation, and so on. The ECG signals corresponding to different arrhythmia types have different signal characteristics, and the arrhythmia detection model is able to determine the corresponding arrhythmia type based on the characteristics of the ECG signal.

According to the above-described arrhythmia detection method provided by this application, the ECG signal to be detected is input into the pre-established arrhythmia detection model, and the detection result including the arrhythmia type can be obtained by the arrhythmia detection model. In comparison with the fact that the traditional convolutional neural network models in the related art can only perform arrhythmia detection on ECG signals with a fixed length thus resulting in significant limitations, the arrhythmia detection model proposed in this application combines the convolutional neural network and the recurrent neural network, and so can detect ECG signals of different lengths depending on the characteristics of the recurrent neural network, thus effectively improving the versatility of arrhythmia detection.

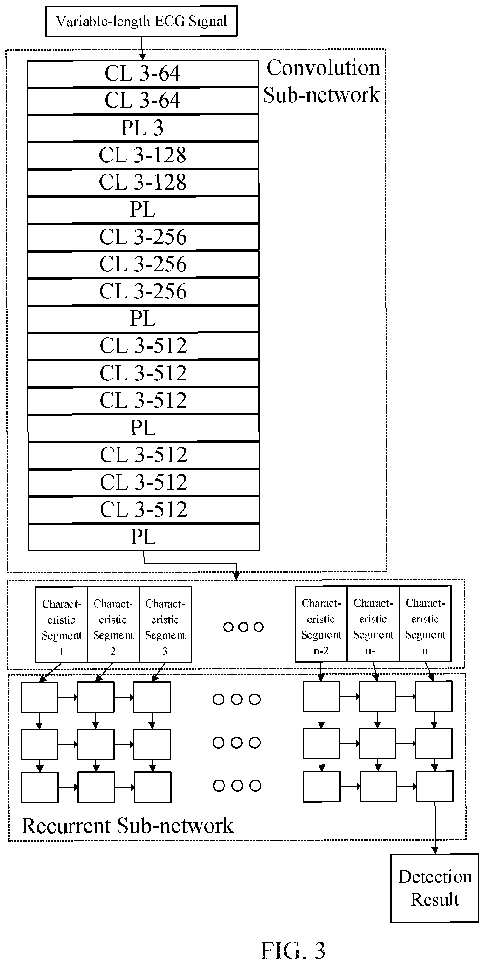

In practical applications, the above-mentioned arrhythmia detection model may also be referred to as a time-stepped convolutional neural network model. In a specific implementation, referring to the schematic diagram of another arrhythmia detection model shown in FIG. 3, the arrhythmia detection model includes a convolution sub-network (that is, the aforementioned convolutional neural network) and a recurrent sub-network (that is, the aforementioned recurrent sub-network).

In practical applications, the convolution sub-network illustrated in FIG. 3 may expose the features of the input signal by iterated convolution and nonlinear transformation based on the input signal, thus generating a temporal sequence composed of multiple characteristic segments. Each characteristic segment corresponds to the feature extracted from a segment of the ECG signal and is input into the recurrent sub-network. The recurrent sub-network may obtain the characteristic segment corresponding to each time point as input by iterating over time, and update the internally saved state of the recurrent sub-network. After the recurrent sub-network finishes reading the full strip of the characteristic temporal sequence, the terminal unit of the recurrent sub-network may output the classification result according to the final state encompassing the information of the entire strip of signal.

For ease of understanding, the working principle of the convolutional neural network in the arrhythmia detection model provided in this embodiment is further elaborated as follows with reference to FIG. 3. The convolutional neural network may include multiple convolutional layers and multiple pooling layers. The convolutional layers may perform convolution operations on the input signal through multiple convolution kernels to generate a signal with more prominent features. The pooling layers may use the maximum pooling operation, that is, taking the maximum value among the input adjacent elements and outputting the same, thereby reducing the size of the data stream. As illustrated in FIG. 3, the convolutional neural network uses a total of 10 convolutional layers and 5 pooling layers. Of the numbers following the term "convolution layer", the former represents the size of the convolution kernel used by this convolution layer, and the latter represents the number of convolution kernels used. Taking the convolutional layer 3-64, 3 represents that the size of the convolution kernel used by this convolutional layer is 3, and 64 represents the number of convolution kernels used by this convolution layer is 64. When performing a convolution operation in the convolution layers, in order to prevent the convolution operation from affecting the signal length, zeros can be filled at both ends of the signal (it is understood that the ECG signal may be represented by a digital sequence, so this specifically means filling zeros at both ends of the digital sequence). In this way, the signal length may be prevented from being changed by the convolutional layer, thus further ensuring the accuracy and reliability of model detection. In practical applications, as the mapping features of the signal points continue to increase due to the increase of the number of convolution kernels, the pooling layers may be used to reduce the size of the number of signal points, thereby maintaining a stable data flow in the model, and avoiding the situation that information may be lost in the process of layer-by-layer transmission due to different expression capabilities between layers. It is to be noted however that the structure of the convolution sub-network shown in FIG. 3 is only one implementation, and may be flexibly changed as required depending on practical applications. Thus, the structure of the convolution sub-network as shown should not be construed as limiting.

This embodiment further provides a specific internal implementation of the convolutional layer, where each convolutional layer includes a convolution unit, a nonlinear transformation unit, and a batch normalization unit that are sequentially connected. In particular, the convolution unit is used to perform a convolution operation, and the nonlinear transformation unit mainly introduces a linear rectification function, which may also be called a rectified linear unit, which is an activation function. The linear rectification function serves as the activation function of the neuron, and defines the nonlinear output result of the neuron after the linear transformation. That is, the linear rectification function is mainly used to introduce nonlinear transformations to the convolutional layer, so that the convolutional neural network has the capability of extraction and complex transformations of the expressed features. During the training process, the batch normalization layer may perform normalization on the received input information, so that the input of each convolutional layer maintains a similar distribution and that the convolution kernel parameters may be updated in a steadier manner.

Further, in this embodiment, the working principle of the recurrent neural network in the arrhythmia detection model is further elaborated as follows. The recurrent neural network may include multiple long- and short-term memory units. In one embodiment, the recurrent neural network may include 3 long- and short-term memory network units. The long- and short-term memory network unit may remember historical information and be used to process temporal sequence data. In actual implementation, each long- and short-term memory unit may include a forget gate structure, an update gate structure, and an output gate structure.

That is, the long- and short-term memory network unit may choose to save or update its internal state variables through three gate structures, the forget gate, the update gate, and the output gate. Through its internal state variables, the long- and short-term memory network unit may save the valid information in the sequence input, and continue to use the information from the earlier locations in the sequence in each state update, so as to achieve the purpose of comprehensive utilization of the information of the entire strip of ECG signal. In one embodiment, the three long- and short-term memory network units have state variables of lengths 128, 32, and 9, respectively. The latter long- and short-term memory network unit may continue to refine and summarize the information contained in the state variable of the former long- and short-term memory network unit. The last long- and short-term memory unit may output the state variable at the last moment as the output of the entire network structure.

The forget gate determines how many of the unit states (i.e., the above state variables) at the previous moment are retained to the current moment. The input gate determines how many of the network's inputs at the current moment are saved to the unit states. The long- and short-term memory network unit uses the output gate to control how many of the unit states are output to the current output value of the long- and short-term memory network unit.

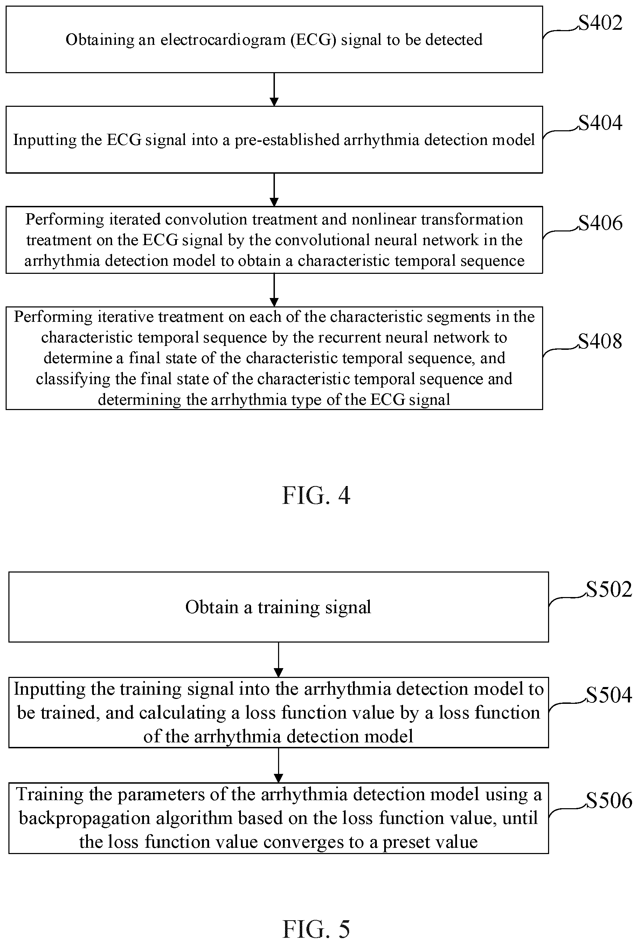

Based on the structure of the above arrhythmia detection model, FIG. 4 shows a flowchart of another arrhythmia detection method, and the method may include the following operations on the basis of FIG. 1.

In S402, the method includes obtaining an electrocardiogram (ECG) signal to be detected.

In S404, the method includes inputting the ECG signal into a pre-established arrhythmia detection model. The arrhythmia detection model may include a convolutional neural network and a recurrent neural network connected in sequence.

In S406, the method may include performing iterated convolution treatment and nonlinear transformation treatment on the ECG signal by the convolutional neural network in the arrhythmia detection model to obtain a characteristic temporal sequence, the characteristic temporal sequence including characteristic segments that are respectively extracted from multiple segments of the ECG signal and that are arranged in chronological order.

In S408, the method may include performing iterative processing on each of the characteristic segments in the characteristic temporal sequence using the recurrent neural network to determine the final state of the characteristic temporal sequence, and classifying the final state of the characteristic temporal sequence and determining the arrhythmia type of the ECG signal. It is to be understood that the purpose of recurrent neural network is to process sequence data. In the traditional convolutional neural network model, the path goes from the input layer to the hidden layer and then to the output layer, the layers are fully connected, while the nodes in each layer are not connected. But such ordinary neural network is powerless for many problems. This is because a lot of information needs to be correlated back and forth. In contrast, for a recurrent neural network, the current output of a sequence is also related to the previous output. In particular, the recurrent neural network may memorize the previous information and apply it to the calculation of the current output, that is, there are connections between adjacent nodes of the layers in the recurrent neural network. Based on this, the recurrent neural network can theoretically process sequence data of any length. Compared with the conventional neural network in the related art that needs to perform feature extraction and classification on the entire strip of signal from the beginning, the recurrent neural network in the arrhythmia detection model provided in this embodiment would store the information of the past ECG signals that have been analyzed. Thus, in each time of calculating the result, only the newly obtained signal needs to be processed, which greatly reduces the amount of computation.

According to the above-described arrhythmia detection method provided by this application, the ECG signal to be detected is input into the pre-established arrhythmia detection model, and the detection result including the arrhythmia type can be obtained through the arrhythmia detection model. In comparison with the fact that the traditional convolutional neural network models in the related art can only perform arrhythmia detection on ECG signals with a fixed length and needs to calculate the entire strip of ECG signal resulting in complex computation, the arrhythmia detection model proposed in this application combines the convolutional neural network and the recurrent neural network, and can detect ECG signals of different lengths depending on the characteristics of the recurrent neural network, thus effectively improving the versatility of arrhythmia detection. Furthermore, it can memorize the information of historical ECG signals. Based on the memorized information, the training of the neural network would require less amounts of computation for each processing section, thereby significantly reducing the required computations and memory space, making it possible to deploy this method on mobile platforms.

Further, this application provides a training process of the arrhythmia detection model. Referring to the flowchart illustrating a training method for an arrhythmia detection model shown in FIG. 5. The training method may include the following steps.

In S502, the training method includes obtain a training signal.

In a specific implementation, an original ECG signal may first be obtained, and then preprocessed to generate a training signal, where the preprocessing may include one or more selected from the group consisting of noise treatment, transverse stretching treatment, transverse compression treatment, and partial masking treatment.

In S504, the training method includes inputting the training signal into the arrhythmia detection model to be trained, and calculating a loss function value by a loss function of the arrhythmia detection model.

In S506, the training method includes training the parameters of the arrhythmia detection model using a backpropagation algorithm based on the loss function value, until the loss function value converges to a preset value.

Through the above method, when the loss function value converges to the preset value, it is determined that the training of the arrhythmia detection model is completed.

For ease of understanding, this embodiment further provides the following training signal generation examples. The foregoing step of preprocessing the original ECG signal to generate the training signal may be performed according to one or more of the following methods.

### (1) Noise Treatment

In actual implementation, a random noise corresponding to the amplitude of a preset signal-to-noise ratio may be added to the original ECG signal to obtain the training signal.

The reason for the noise treatment of the training signal is that when actually collecting the ECG signal of a person to be tested, the collected ECG signal must contain noise (white noise is taken as an example for explanation below), because of the limitations of the collection. In order that the arrhythmia detection model better performs arrhythmia detection on ECG signals containing white noise in practical applications, this embodiment may perform noise treatment on the original ECG signal (i.e., the original pure ECG signal free of noise) when training the arrhythmia detection model. For example, after estimating the signal-to-noise ratio, some random white noise corresponding to the amplitude of the signal-to-noise ratio could be added to the original ECG signal, thereby generating a new segment of signal (i.e., training signal) of the original ECG signal interfered by different white noises. Training the arrhythmia detection model using the training signals containing noise may enable the arrhythmia detection model not to be interfered by the noise of the actual collected ECG signal of the person to be detected during practice, which helps to improve the accuracy and reliability of the detection results of the arrhythmia detection model.

### (2) Transverse Stretching Treatment and/or Transverse Compression Treatment

In specific implementation, the original ECG signal may be subjected to transverse stretching treatment and/or transverse compression treatment according to a preset ratio to obtain the training signal.

The reason of performing the transverse stretching treatment and/or transverse compression treatment is that the heart rate of the human body fluctuates within a certain range, depending on the current physical state of the individual. In addition, different individuals may also have different heart rates. Therefore, by transversely stretching/compressing the signal by a factor within a certain range (i.e., a preset ratio), namely, by stretching/compressing the original ECG signal, the ECG signals under various heart rates (that is, the training signals) can be generated. By training the arrhythmia detection model using the ECG signals under various heart rates, the detection effect of the arrhythmia detection model for the ECG signals under various heart rates can be improved, so that the arrhythmia detection model can focus more on the global features that are not affected by heart rate, such as waveform characteristics and heart rate variability (HRV).

### (3) Partial Masking Treatment

In specific implementation, a signal segment with a duration lower than the preset duration may be randomly selected from the original ECG signal, and then the selected signal segment in the original ECG signal may be set to zero to obtain the training signal.

The reason for the partial masking treatment of the training signal is that the signal strength of the tested person may fluctuate or even the lead may fall off when actually collecting the ECG signal of the tested person. The ideal model must be able to eliminate the influence of partial signal loss as much as possible, so as to autonomously extract features from the valid signal segments. In view of this, the randomly selected segment may be set to zeros in the original ECG signal to create the effect of partial signal loss. For example, a segment of no longer than 1.5 seconds may be randomly selected from each ECG signal, and this signal segment may be set to zeros, thereby generating various lead-off signals. By training the arrhythmia detection model using partially missing ECG signals thus improving the detection effect of the arrhythmia detection model on partially missing ECG signals, the arrhythmia detection model can be prevented from only focusing on some significant signal segments in the ECG signal, thereby facilitating the detection model to extract signal features more comprehensively and improving the detection effect.

The foregoing methods of generating the training signal may enhance the training data of the arrhythmia detection model. By transforming the original ECG signal to a certain extent, a new ECG signal that is roughly attributable to a unified data distribution with the original ECG signal is generated. Such transformations are mainly intended for the purpose of simulating some inherent characteristics involving randomness in the actually collected ECG signal. The enhanced data (i.e., the training signal) may greatly increase the learning accuracy during the model training process, so that the model maybe better trained and the arrhythmia detection effect of the model maybe improved.

In actual training, the arrhythmia detection model provided in this embodiment can support the end-to-end training method, that is, it can better couple the convolutional neural network with the recurrent neural network, so that the two networks can organically merge and promote each other.

In order to further improve the stability and reliability of the arrhythmia detection model, the training process of the arrhythmia detection model may adopt one or more stable training methods selected from the group consisting of a regularization method, a network connection structure random removal method, and a label interference method. For ease of understanding, the above training methods are described in detail as follows.

### (1) Regularization Method

The neural network model usually has many parameters, making it prone to overfitting. In view of this, a regularization method may be used to prevent overfitting in this embodiment when training the model. The regularization method can adopt the L1 regularization method or the L2 regularization method. By constraining the internal parameters of the model, it is possible to effectively control the situation where some parameters are updated to dominate the trend of the entire model. In practical applications, a certain proportion of the sum of squares of all parameters may be added to the loss function during model training to establish soft constraints on internal parameters, thereby reducing the possibility of excessive model parameters and enabling stable training of the model.

### (2) Network Connection Structure Random Removal Method

The network connection structure random removal method may be to randomly remove fixed-size parts in the neural network structure. For example, in the connections of a previous convolutional layer to a next convolutional layer in the neural network, some filters of the convolutional layer may be controlled to not operate. By breaking the original network structure in this way, different smaller neural network structures may be mainly trained during each training instance. Such random connection combination may free the model from strong dependence on partial connections, so as to make more effective use of the overall model parameters thus obtaining a more stable and reliable model.

### (3) Label Interference Method

The label interference method may be to moderately randomly introduce wrong labels when training the model. For example, the correct label corresponding to the training signal A is B, but during training the training signal A may be labeled with a label C. Through this wrong label interference method, the model can be based on a smaller data set every time it is trained, so that the trained model approximates the average of the models generated by multiple different sampled data sets. Such kind of averaging helps to obtain a more stable model.

The training method of the above-described arrhythmia detection model provided in this embodiment is helpful for stably training the model, so as to obtain an arrhythmia detection model that can obtain accurate and reliable detection results in practical applications.

Furthermore, in the implementation of real-time processing of this model, the accuracy and real-time performance can be flexibly balanced as the application requirements change. In one implementation where the model provided in this embodiment is applied, to achieve the best accuracy, the data distribution of the ECG signal maybe kept consistent with the training signal. In particular, when training the model, the convolutional layers of the model may be made to fill zeros at both ends of the data sequence corresponding to the training signal, while most of the data except for the data at the two ends may be pure data without needing the zero filling operation. In order to achieve the best real-time performance, the model maybe allowed to fill in zeros at both ends of the data sequence corresponding to the training signal in all convolutional layer calculations, so that the convolutional layers no longer affect the data length required for a single prediction update. It is to be understood that the ECG signal is actually a combination of multiple numbers, and a string of ECG signal is actually a digital sequence, and the purpose of the above zero-filling is to facilitate the convolution operation and prevent the signal length from being affected by the convolution operation.

As described supra, the foregoing arrhythmia detection method provided in this embodiment uses an arrhythmia detection model (also called a time-stepped convolutional neural network), which combines the convolutional neural network and the recurrent neural network, and is applicable for ECG signals of different lengths (i.e., compatible with different variable-length ECG signals), and is able to automatically extract and comprehensively utilize the features in the ECG signals. In addition, the arrhythmia detection model can be applied to real-time processing. Because the recurrent neural network is used to store the information of the past ECG signals that have been analyzed, each time the result is calculated, it only needs to process the newly obtained signal. Compared with other neural network models that need to perform feature extraction and classification of the entire strip of signal from the very beginning, the arrhythmia detection model provided in this embodiment can greatly reduce the amounts of computation, support incremental real-time processing, and can be used in a real-time processing environment. Therefore, compared with the traditional convolutional neural network models, the amounts of memory required by the model provided in this embodiment are significantly reduced, making it possible to deploy the method on mobile platforms.

Further, the above-described arrhythmia detection model can fully and comprehensively extract local features (such as a single waveform in the ECG signal) and global features (such as heart rate and heart rate variability) from the variable-length ECG signals. Because of its superior detection performance, low memory overhead, low computational overhead during real-time processing, etc., it has good applicability in the technical application fields of ECG signal automatic analysis, such as hospital monitoring and patient self-monitoring.

In order to further verify the effect of the arrhythmia detection model provided in this embodiment, this application provides the following experimental data. In distinguishing between 8 types of arrhythmias from normal heart rhythms, this application achieves an average accuracy rate of up to 77.3%. Compared with the best results of the VGG16 model (i.e., an existing detection model) under several different input lengths, the time-stepped convolutional neural network (i.e., the arrhythmia detection model) proposed in this application) reduces the error rate by more than 6%, and achieves significant accuracy improvements in specific arrhythmia types, such as atrial premature contraction and ventricular premature contraction, by more than 22% and 13%, respectively. The specific results are shown in Table 1.

**Table 1 Comparison of Arrhythmia Classification Performance Between Traditional Convolutional Neural Network and Time-stepped Convolutional Neural Network**

| Type | Accuracy | | | | Number of Test Cases |
|---|---|---|---|---|---|
| | VGG16 (6-second input) | VGG16 (12-secondinput) | VGG16 (3 0-secondinput) | Time-stepped CNN | |
| Normal | 0.694 | 0.708 | 0.659 | 0.759 | 91 |
| Atrial fibrillation | 0.811 | 0.787 | 0.787 | 0.807 | 110 |
| First degree atrioventricular block | 0.812 | 0.754 | 0.800 | 0.855 | 70 |
| Left bundle branch block | 0.714 | 0.727 | 0.634 | 0.844 | 21 |
| Right bundle branch block | 0.824 | 0.783 | 0.802 | 0.837 | 169 |
| Atrial premature contraction | 0.378 | 0.468 | 0.319 | 0.606 | 57 |
| Ventricular premature contraction | 0.576 | 0.605 | 0.491 | 0.712 | 65 |
| ST depression | 0.667 | 0.698 | 0.687 | 0.742 | 83 |
| ST elevation | 0.483 | 0.438 | 0.214 | 0.556 | 19 |
| Average | | | | | |
| Accuracy | 0.717 | 0.709 | 0.678 | 0.775 | 685 |
| Sensitivity | 0.710 | 0.706 | 0.675 | 0.775 | |
| Accuracy | 0.711 | 0.706 | 0.675 | 0.773 | |

Furthermore, the parameters of the time-stepped convolutional neural network model proposed in this application can be calculated. By estimating the parameters of the convolutional layers, we conclude that the quantity of parameters of the time-stepped convolutional network model is about half of that of the VGG16 model. The specific estimation process is illustrated in Table 2 below.

Benefiting from the design of removing the fully connected layer, the time-stepped convolutional network model proposed in this application greatly reduces the amount of required parameters through parameter multiplexing in the convolutional layers and the long- and short-term memory network.

**Table 2 Comparison of Quantities of Parameters of Traditional Convolutional Neural Network and Time-stepped Convolutional Neural Network**

| Layer | | Parameter Calculation Formula | | Quantity of Parameters | |
|---|---|---|---|---|---|
| VGG16 | Time-stepped CNN | VGG16 | Time-stepped CNN | VGG16 | Time-stepped CNN |
| Convolutional Layer 3-64 | | 12×64×3 | | 2304 | |
| Convolutional Layer 3-64 | | 64×64×3 | | 12,288 | |
| Convolutional Layer 3-128 | | 64×128×3 | | 24,576 | |
| Convolutional Layer 3-128 | | 128×128×3 | | 49,152 | |
| Convolutional Layer 3-256 | | 128×256×3 | | 98,304 | |
| Convolutional Layer 3-256 | | 256×256×3 | | 196,608 | |
| Convolutional Layer 3-256 | | 256×256×3 | | 196,608 | |
| Convolutional Layer 3-512 | | 256×512×3 | | 393,216 | |
| Convolutional Layer 3-512 | | 512×512×3 | | 786,432 | |
| Convolutional Layer 3-512 | | 512×512×3 | | 786,432 | |
| Convolutional Layer 3-512 | | 512×512×3 | | 786,432 | |
| Convolutional Layer 3-512 | | 512×512×3 | | 786,432 | |
| Convolutional Layer 3-512 | | 512×512×3 | | 786,432 | |
| Fully Connected Layer 1024 | Long- and Short-term Memory 128 | 512×12×1024 | (512+128)×128×4 | 6,291,456 | 327,680 |
| Fully connected | Long- and Short-term | 1024×256 | (128+32)×32×4 | 262,144 | 20,480 |
| layer 256 | Memory 32 | | | | |
| Fully connected layer 9 | Long- and Short-term Memory 9 | 256×9 | (32+9)×9×4 | 2,304 | 1,476 |
| Total | | | | 11,461,120 | 5,254,852 |

As can be seen from Table 2, the quantity of parameters of the traditional convolutional neural network VGG16 is 11,461,120, while that of the time-stepped convolutional neural network provided by this application is only 5,254,852, which is significantly less than the parameters of the traditional network model.

In view of the above, the arrhythmia detection method provided by this application has the following prominent advantages by using the time-stepped convolutional neural network.
(1) It can be applied to variable-length ECG signals, and is suitable for various types of ECG signal databases.
(2) It doesn't require explicit division of the ECG signal, and can comprehensively utilize the global and local features in the ECG record.
(3) Through the design that only includes the convolutional neural network and the recurrent neural network, the number of parameters is greatly reduced, thus saving the memory cost required to deploy the model.
(4) It supports real-time processing, and can greatly reduce the amounts of computation in real-time processing solutions, because the recurrent neural network unit is used to store the previous state information.

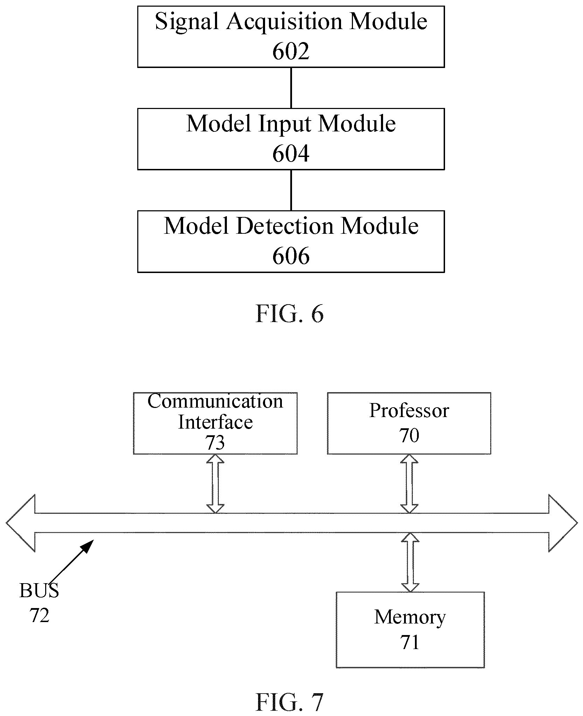

Corresponding to the foregoing arrhythmia detection method, an embodiment of the present application further provides an arrhythmia detection apparatus, which may be configured on the processor side of an electronic device. Referring to the block diagram of an arrhythmia detection apparatus illustrated in FIG. 6, the arrhythmia detection apparatus may include a signal acquisition module 602, a model input module 604, and a model detection module 606.

The signal acquisition module 602 is configured to obtain an electrocardiogram (ECG) signal to be detected.

The model input module 604 is configured for inputting the ECG signal into a pre-established arrhythmia detection model, where the arrhythmia detection model may include a convolutional neural network and a recurrent neural network connected in sequence. In actual implementation, the convolutional neural network may include a plurality of convolutional layers and a plurality of multiple pooling layers. Each of the convolutional layers may include a convolution unit, a nonlinear transformation unit, and a batch normalization unit that are connected in sequence. The recurrent neural network may include a plurality of long- and short-term memory units, where each of the long- and short-term memory units may include a forget gate structure, an update gate structure, and an output gate structure.

The model detection module 606 may be configured for detecting the ECG signal by the arrhythmia detection model to obtain a detection result corresponding to the ECG signal, the detection result including the arrhythmia type.

According to the above-described arrhythmia detection apparatus provided by this application, the ECG signal to be detected is input into the pre-established arrhythmia detection model, and the detection result including the arrhythmia type can be obtained through the arrhythmia detection model. In comparison with the fact that the traditional convolutional neural network models in the related art can only perform arrhythmia detection on ECG signals with a fixed length thus resulting in significant limitations, the arrhythmia detection model proposed in this application combines the convolutional neural network and the recurrent neural network, and so can detect ECG signals of different lengths depending on the characteristics of recurrent neural networks, thus effectively improving the versatility of arrhythmia detection.

In one embodiment, the above-described model detection module includes a feature extraction unit and a type determination unit.

The feature extraction unit is configured for performing feature extraction on the input ECG signal through the convolutional neural network to obtain feature information of the electrocardiogram signal, and inputting the feature information into the recurrent neural network.

The type determination unit is configured for classifying the input feature information by the recurrent neural network to determine the arrhythmia type of the ECG signal.

The feature extraction unit may specifically be configured for performing iterated convolution treatment and nonlinear transformation treatment on the ECG signal by the convolutional neural network to obtain a characteristic temporal sequence, the characteristic temporal sequence including multiple characteristic segments that are respectively extracted from multiple segments of the ECG signal and that are arranged in chronological order.

The type determination unit may specifically be configured for performing iterative processing on each of the characteristic segments in the characteristic temporal sequence using the recurrent neural network to determine the state of the characteristic temporal sequence, and classifying the final state of the characteristic temporal sequence and determining the arrhythmia type of the ECG signal.

Further, this embodiment also provides a training method for the arrhythmia detection model. Based on this, the above-described arrhythmia detection apparatus may further include a training signal acquisition module, a loss value calculation module, and a training module that are connected in sequence.

The training signal acquisition module is configured to obtain an electrocardiogram (ECG) signal to be detected.

The loss value calculation module is configured for inputting the training signal into the arrhythmia detection model to be trained, and calculating the loss function value through the preset loss function of the arrhythmia detection model.

The training module is configured for training the parameters of the arrhythmia detection model using a backpropagation algorithm based on the loss function value, until the loss function value converges to a preset value.

The training signal acquisition module may specifically be configured for obtaining an original ECG signal, and preprocessing the original ECG signal to generate a training signal, the preprocessing including one or more selected from the group consisting of noise treatment, transverse stretching treatment, transverse compression treatment, and partial masking treatment.

When performing noise treatment on the original ECG signal, random, a random noise corresponding to the amplitude of a preset signal-to-noise ratio may be added to the original ECG signal to obtain the training signal.

When performing transverse stretching treatment and/or transverse compression treatment on the original ECG signal, the original ECG signal may be subjected to transverse stretching treatment and/or transverse compression treatment according to a preset ratio to obtain the training signal.

When performing partial masking treatment on the original ECG signal, a signal segment with a duration lower than the preset duration may be randomly selected from the original ECG signal, and then the selected signal segment in the original ECG signal may be set to zero to obtain the training signal.

The implementation principles and technical effects of the arrhythmia detection apparatus provided in this embodiment are the same as those of the foregoing embodiment. Thus, for brevity of description, refer to the corresponding content in the foregoing method embodiment for the parts not mentioned in this apparatus embodiment.

Further, an embodiment of the present application further provides an electronic device that includes a memory and a processor, where the memory is configured to store a program that supports the processor to execute any one of the foregoing arrhythmia detection methods, and the processor is configured to execute the program stored in the memory. The electronic device may be a computer, a mobile phone, or a wearable heart monitoring device.

In addition, an embodiment of the present application further provides a computer storage medium configured to store computer software instructions used by any of the aforementioned arrhythmia detection apparatuses.

This application provides an electronic device, referring to the schematic diagram of an electronic device shown in FIG. 7. The electronic device includes a processor 70, a memory 71, a bus 72, and a communication interface 73. The processor 70, the communication interface 73, and the memory 71 are coupled by the bus 72. The processor 70 is used to execute executable modules (such as a computer program) stored in the memory 71.

The memory 71 may include a high-speed random access memory (RAM), or may also include a non-volatile memory, such as at least one disk memory.

The communicative connection between this system network element and at least one other network element may be realized through at least one of the communication interface 73 (wired or wireless), where the Internet, a wide area network, a local network, a metropolitan area network, etc. may be used.

The bus 72 may be an ISA bus, PCI bus, EISA bus, or the like. The bus may be divided into an address bus, a data bus, a control bus, or the like. For ease of representation, only one bidirectional arrow is used in FIG. 7, but it does not mean that there is only one bus or one type of bus.

The memory 71 is used to store a program. The processor 70 executes the program after receiving an execution instruction. The flow-defined methods executed by the apparatuses that are disclosed in any of the foregoing embodiments of the present application can be applied to the processor 70 or implemented by the processor 70.

The processor 70 may be an integrated circuit chip having signal processing capabilities. In the implementation, the steps of the foregoing methods can be fulfilled by hardware integrated logic circuits in the processor 70 or by instructions in the form of software. The aforementioned processor 70 may be a general-purpose processor, including a central processing unit (CPU), a network processor (NP), or the like. It may also be a digital signal processor (DSP), an application specific integrated circuit (ASIC), a field-programmable gate array (FPGA), or other programmable logic devices, discrete gates, transistor logic devices, or discrete hardware components. They can implement or execute the various methods, steps, and logic block diagrams disclosed in this application. The general-purpose processor may be a microprocessor or any conventional processor or the like. The steps of the methods disclosed in this application may be directly embodied as being executed by a hardware decoding processor, or executed by a combination of hardware and software modules in the decoding processor. Software modules may be located in a mature storage medium in the field to which this application pertains, such as random access memory, flash memory, read-only memory, programmable read-only memory, electrically erasable programmable memory, or registers. The storage medium is located in the memory 71, and the processor 70 reads the information in the memory 71 and performs the steps of the foregoing methods in conjunction with its hardware.

Those skilled in the art will be able to clearly appreciate that, for convenience and brevity of description, they can refer to the corresponding processes in the foregoing embodiments for the specific working processes of the electronic device described above, which will therefore not be detailed here again.

The computer program products of the arrhythmia detection method and apparatus, electronic device, and computer storage medium provided by this application may include a computer readable storage medium storing program code, where the instructions included in the program code may be used to execute the methods in the foregoing method embodiments, referring to the specific implementation of the method embodiments for details, which will not be described here again.

As used herein, terms "installed on", "mounted on", "connected to", "coupled to", "connected with", and "coupled with" should be understood in a broad sense unless otherwise specified and defined. For example, they may indicate a fixed connection, a detachable connection, or an integral connection. They may denote a mechanical connection, or an electrical connection. They may denote a direct connection, a connection through an intermediate, or an internal connection between two elements. For those of ordinary skill in the art, the specific meanings of the above terms as used in the present application can be understood on a case-by-case basis.

If the function is implemented in the form of a software functional unit and sold or used as an independent product, it may be stored in a computer readable storage medium. Based on such an understanding, the essential technical of the present application or the part that contributes to the related art, or a part of the technical solution may be embodied in the form of a software product. The computer software product may be stored in a storage medium, which may include multiple instructions configured to cause a computing device(e.g., a personal computer, a server, or a network device, etc.) to execute all or part of the steps of the methods described in various embodiment of this application. The aforementioned storage medium may include a U disk, a mobile hard drive, a read-only memory (ROM), a random access memory (RAM), a magnetic disk, an optical disk, or various other media that can store program code.

Finally, it should be noted that the foregoing embodiments merely represent some specific implementations of the present application, and are used for the mere purpose of illustrating, rather than limiting, the technical solutions of the present application, and so the scope of protection of this application will not be limited to these specific embodiments. The scope of protection of this application is not limited to this. Although this application has been described in detail in connection with the foregoing embodiments, a person having ordinary skill in the art should understand that any person skilled in the art should be able to modify or easily think of changes to the technical solutions described in the foregoing embodiments without departing from the technical scope disclosed in this application, or equivalently replace some of the technical features. These modifications, changes, or replacements do not render the substance of the corresponding technical solutions to deviate from the spirit and scope of the technical solutions of this example, and thus shall be covered within the scope of protection of this application. Therefore, the scope of protection of this application shall be defined by the scope of protection of the appended claims.

### Industrial Applicability

According to the technical solution of this application, an arrhythmia detection model including a convolutional neural networks and a recurrent neural network that are connected in sequence is used for arrhythmia detection on ECG signals of different lengths, thereby effectively improving the versatility of arrhythmia detection.

## Claims

1. An arrhythmia detection method, which is executed by a processor of an electronic device and comprises:
Obtainingan electrocardiogram (ECG) signal to be detected;
Inputting the ECG signal into a pre-established arrhythmia detection model, the arrhythmia detection model comprising a convolutional neural network (CNN) and a recurrent neural network (RNN) that are connected in sequence; and
Detecting the ECG signal by the arrhythmia detection model to obtain a detection result corresponding to the ECG signal, the detection result comprising an arrhythmia type.

2. The arrhythmia detection method as recited in claim 1, wherein the operation of "detecting the ECG signal by the arrhythmia detection model to obtain a detection result corresponding to the ECG signal" comprises:
performing feature extraction on the input ECG signal by the CNN to obtain feature information of the ECG signal, and inputting the feature information into the RNN; and
classifying the input feature information by the RNN to determine the arrhythmia type of the ECG signal.

3. The arrhythmia detection method as recited in claim 2, wherein the operation of "performing feature extraction on the input ECG signal by the CNN to obtain feature information of the ECG signal" comprises:
Performing iterated convolution treatment and nonlinear transformation treatment on the ECG signal by the CNN to obtain a characteristic temporal sequence, the characteristic temporal sequence comprising characteristic segments that are respectively extracted from a plurality of segments of the ECG signal and that are arranged in chronological order.

4. The arrhythmia detection method as recited in claim 2, wherein the operation of "classifying the input feature information by the RNN to determine the arrhythmia type of the ECG signal" comprises:
performing iterative treatment on each of the characteristic segments in the characteristic temporal sequence by the RNN to determine a final state of the characteristic temporal sequence, and classifying the final state of the characteristic temporal sequence thus determining the arrhythmia type of the ECG signal.

5. The arrhythmia detection method as recited in claim 1, wherein a training process of the arrhythmia detection model comprises:
obtain a training signal;
inputting the training signal into the arrhythmia detection model to be trained, and calculating a loss function value by a loss function of the arrhythmia detection model; and
training parameters of the arrhythmia detection model using a backpropagation algorithm based on the loss function value, until the loss function value converges to a preset value.

6. The arrhythmia detection method as recited in claim 5, wherein the operation of "obtaining a training signal" comprises:
obtaining an original electrocardiogram (ECG) signal;
preprocessing the original ECG signal to generate the training signal, the preprocessing comprising one or more selected from the group consisting of noise treatment, transverse stretching treatment, transverse compression treatment, and partial masking treatment.

7. The arrhythmia detection method as recited in claim 6, wherein the preprocessing comprises noise treatment, and wherein the operation of "preprocessing the original ECG signal to generate the training signal" comprises:
adding a random noise corresponding to an amplitude of a preset signal-to-noise ratio to the original ECG signal to obtain the training signal.

8. The arrhythmia detection method as recited in claim 6, wherein the preprocessing comprises transverse stretching treatment and/or transverse compression treatment, and wherein the operation of "preprocessing the original ECG signal to generate the training signal" comprises:
subjecting the original ECG signal to transverse stretching treatment and/or transverse compression treatment according to a preset ratio to obtain the training signal.

9. The arrhythmia detection method as recited in claim 6, wherein the preprocessing comprises partial masking treatment, and wherein the operation of "preprocessing the original ECG signal to generate the training signal" comprises:
randomly selecting a signal segment with a duration lower than a preset duration from the original ECG signal; and
setting the selected signal segment in the original ECG signal to zeros to obtain the training signal.

10. The arrhythmia detection method as recited in claim 5, wherein the training process of the arrhythmia detection model adopts one or more stable training methods selected from the group consisting of a regularization method, a network connection structure random removal method, and a label interference method.

11. The arrhythmia detection method as recited in any one of claims 1 to 10, wherein the CNN comprises a plurality of convolutional layers and a plurality of pooling layers, wherein each of the plurality of convolutional layers comprises a convolution unit, a nonlinear transformation unit, and a batch normalization unit that are connected in sequence;
the RNN comprises a plurality of long- and short-term memory units, wherein each of the long- and short-term memory units comprises a forget gate structure, an update gate structure, and an output gate structure.

12. An arrhythmia detection apparatus, which is configured on a processor side of an electronic device and comprises:
a signal acquisition module, configured for obtaining an electrocardiogram (ECG) signal to be detected;
a model input module, configured for inputting the ECG signal into a pre-established arrhythmia detection model, the arrhythmia detection model comprising a convolutional neural network (CCN) and a recurrent neural network (RNN) that connected in sequence; and
a model detection module, configured for detecting the ECG signal through the arrhythmia detection model to obtain a detection result corresponding to the ECG signal, the detection result comprising an arrhythmia type.

13. The arrhythmia detection apparatus as recited in claim 12, wherein the model detection module comprises:
a feature extraction unit, configured for performing feature extraction on the input ECG signal by the CNN to obtain feature information of the ECG signal, and inputting the feature information into the RNN; and
a type determination unit, configured for classifying the input feature information by the RNN to determine the arrhythmia type of the ECG signal.

14. The arrhythmia detection apparatus as recited in claim 13, wherein the feature extraction unit is configured for:
performing iterated convolution treatment and nonlinear transformation treatment on the ECG signal by the CNN to obtain a characteristic temporal sequence, the characteristic temporal sequence comprising a plurality of characteristic segments that are respectively extracted from a plurality of segments of the ECG signal and that are arranged in chronological order.

15. The arrhythmia detection apparatus as recited in claim 13, wherein the type determination unit is configured for:
performing iterative treatment on each of the plurality of characteristic segments in the characteristic temporal sequence by the RNN to determine a final state of the characteristic temporal sequence, and classifying the final state of the characteristic temporal sequence and determining the arrhythmia type of the ECG signal.

16. The arrhythmia detection apparatus as recited in claim 12, further comprising:
a training signal acquisition module, configured to obtain a training signal;
a loss value calculation module, configured for inputting the training signal into the arrhythmia detection model to be trained, and calculating a loss function value through a preset loss function of the arrhythmia detection model; and
a training module, configured for training parameters of the arrhythmia detection model through a backpropagation algorithm based on the loss function value, until the loss function value converges to a preset value.

17. The arrhythmia detection apparatus as recited in claim 16, wherein the training signal acquisition module is configured for:
obtaining an original electrocardiogram (ECG) signal; and
preprocessing the original ECG signal to generate the training signal, the preprocessing comprising one or more selected from the group consisting of noise treatment, transverse stretching treatment, transverse compression treatment, and partial masking treatment.

18. The arrhythmia detection apparatus as recited in claim 16, wherein the CNNcomprises a plurality of convolutional layers and a plurality of pooling layers, wherein each of the plurality of convolutional layers comprises a convolution unit, a nonlinear transformation unit, and a batch normalization unit that are connected in sequence;
the RNN comprises a plurality of long- and short-term memory units, wherein each of the long- and short-term memory units comprises a forget gate structure, an update gate structure, and an output gate structure.

19. An electronic device, comprising a memory and a processor, wherein the memory is configured to store a program that supports the processor to execute the arrhythmia detection method as recited in any one of claims 1 to 11, and the processor is configured to execute the program stored in the memory.

20. A computer storage medium, configured for storing computer software instructions used by the arrhythmia detection apparatus as recited in any one of claims 12 to 18.
